# EUROPÄISCHE PATENTSCHRIFT

(11) **EP 2 367 557 B1**
(45) Veröffentlichungstag und Bekanntmachung des Hinweises auf die Patenterteilung: **31.10.2012**
(21) Anmeldenummer: 09778624.8
(22) Anmeldetag: 21.09.2009
(51) Int. Cl.: A61K 36/35, A61P 1/02, A61K 31/164, A61K 31/185, A61K 36/09, A61K 36/48, A61K 36/68, A61K 36/898, A61K 36/732

(54) **ZUSAMMENSETZUNG ZUR BEHANDLUNG VON ENTZÜNDLICHEN ERKRANKUNGEN DES MUND- UND RACHENRAUMS**
COMPOSITION FOR THE TREATMENT OF INFLAMMATORY DISEASES OF THE ORAL AND PHARYNGEAL CAVITY
COMPOSITION POUR LE TRAITEMENT DE MALADIES INFECTIEUSES DE LA CAVITÉ BUCCALE ET PHARYNGIENNE

(30) Priorität: 22.12.2008 DE 202008016832 U
(43) Veröffentlichungstag der Anmeldung: 28.09.2011
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE)
(74) Vertreter: Gesthuysen, von Rohr & Eggert
(86) Internationale Anmeldenummer: PCT/EP2009/006791
(87) Internationale Veröffentlichungsnummer: WO 2010/083855

(56) Entgegenhaltungen:
- EP-A1- 1 944 023
- WO-A2-2008/011556
- US-A1- 2003 166 732
- DATABASE WPI Week 200425 Thomson Scientific, London, GB; AN 2004-262859 XP002559327 & JP 2004 091349 A (FANKERU KK) 25. März 2004 (2004-03-25)

## Beschreibung

Die vorliegende Erfindung betrifft das medizinisch-pharmazeutische Gebiet der Therapie von entzündlichen Erkrankungen des Mund- und Rachenraums.

Insbesondere betrifft die vorliegende Erfindung eine Zusammensetzung zur Behandlung von entzündlichen Erkrankungen des Mund- und Rachenraums sowie deren Verwendung.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf.

Nicht beschränkende Beispiele für derartige entzündliche Erkrankungen des Mundraums sowie des Hals-/Rachenraums sind z. B. Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Auch Entzündungen im Bereich der Mundhöhle, wie z. B. Stomatitis, Gingivitis, Mundschleimhautläsionen oder dergleichen, zählen zu den vorgenannten Erkrankungen des Mund- und Rachenraums.

Für weitergehende diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden.

Der Begriff der entzündlichen Erkrankungen des Mund- und Rachenraums ist somit sehr weit zu verstehen und umfaßt sämtliche entzündlichen Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im allgemeinen durch topische und gegebenenfalls zusätzliche systemische Therapie in schwerwiegenderen Fällen. Während in schwereren Fällen systemisch Antibiotika verabreicht werden, kommen unterstützend und in leichteren Fällen unter Umständen alleinig zur topischen, symptomatischen Behandlung oftmals Antiseptika und entzündungshemmende Stoffe (so z. B. Antiphlogistika, antiinflammatorisch wirkende Mittel, Lokalantibiotika etc.) zum Einsatz, welche beispielsweise in Form von Rachenspülungen, Sprays oder Lutschtabletten verabreicht werden können.

Als Wirkstoff zur topischen Behandlung von entzündlichen Prozessen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, daß bei hoher Dosierung und übermäßigem Verzehr Magen-Darm-Beschwerden, Atemnot sowie eine vermehrte Methämoglobinbildung, insbesondere bei Kindern, auftreten kann.

Des weiteren hat sich als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") bewährt, welches beispielsweise als Spray oder in Form von Spül- bzw. Gurgellösungen appliziert werden kann. Bei längerer Anwendung und starker Dosierung kann es auch hier zu Magen-Darm-Beschwerden und darüber hinaus zu Geschmacksirritationen kommen.

Des weiteren kommen Wirkstoffe natürlichen Ursprungs zum Einsatz, so z. B. sogenannte Schleimdrogen oder deren Extrakte, wie z. B. Isländisches Moos (*Lichen islandicus*). Derartige Präparate bieten aber nicht immer den gewünschten Therapieerfolg.

So betrifft die EP 1 944 023 A1 eine pharmazeutische Zusammensetzung, welche einen Extrakt aus Süßholzwurzel und Eichenrinde in Kombination mit Dexpanthenol umfasst.

Weiterhin betrifft die WO 2008/011556 A2 ein Verfahren zur Behandlung bzw. zur Verbesserung einer Indikation einer Schleimhaut oder eines angrenzenden Gewebes, wobei das Verfahren die periodische Applikation einer Spülung auf das in Rede stehende Gewebe umfasst, wobei die Spülung eine bioaktive Kräuterkomponente auf Basis von *Sambucus nigra, Centella asiatica* oder *Echinacea purpurea* einerseits und ein antimikrobielles Mittel auf Basis eines oberflächenaktiven quarternären Ammoniumsalzes aufweist.

Die US 2003/0166732 A1 betrifft die Verwendung von Ambroxol und dessen pharmakologisch aktiven Salzen zur Herstellung einer pharmazeutischen Zusammensetzung zur Behandlung von schmerzhaften Veränderungen der Mund- bzw. Rachenhöhle.

Darüber hinaus betrifft die JP 2004 091349 eine Zusammensetzung auf pflanzlicher Basis, welche neben einer Vielzahl anderer Substanzen auch Holunder enthalten kann. Die Zusammensetzung soll zur Vorbeugung und Behandlung von Infektionskrankheiten eingesetzt werden.

Der vorliegenden Erfindung liegt daher die Aufgabe zugrunde, eine Zusammensetzung, insbesondere pharmazeutische Zusammensetzung, bereitzustellen, welche sich zur effizienten, insbesondere topischen bzw. lokalen Behandlung von entzündlichen Erkrankungen des Mund- und Rachenraums (d. h. also von entzündlichen Erkrankungen des Hals-/Rachenraums und der Mundhöhle) eignet und insbesondere die zuvor geschilderten Nachteile des Standes der Technik zumindest weitgehend vermeidet oder wenigstens abschwächt.

Zur Lösung der zuvor beschriebenen Aufgabe schlägt die vorliegende Erfindung - gemäß einem ersten Erfindungsaspekt - eine Zusammensetzung, insbesondere eine pharmazeutische Zubereitung in Form einer flüssigen Dosierung, nach Anspruch 1 vor; weitere vorteilhafte Ausgestaltungen sind Gegenstand der diesbezüglichen Unter- und Nebenansprüche.

Weiterer Gegenstand der vorliegenden Erfindung - gemäß einem zweiten Erfindungsaspekt - ist ein Applikator, vorzugsweise für die Sprühapplikation, gemäß Anspruch 13, welcher eine Zusammensetzung nach der vorliegenden Erfindung enthält.

Schließlich ist Gegenstand der vorliegenden Erfindung - gemäß einem dritten Erfindungsaspekt - die erfindungsgemäße Verwendung einer Zusammensetzung nach der vorliegenden Erfindung gemäß Anspruch 14; weitere vorteilhafte Ausgestaltungen dieses Erfindungsaspekts sind Gegenstand des diesbezüglichen Unteranspruchs.

Die Anmelderin hat nun überraschenderweise herausgefunden, daß das zuvor geschilderte Problem dadurch gelöst werden kann, daß man (a) mindestens eine Schleimdroge, insbesondere deren Extrakt, (b) Dexpanthenol (Panthenol, Pantothenol) und/oder Pantothensäure oder deren Derivate, insbesondere Ester oder Salze, und (c) Holunder, insbesondere Holunderblüten, insbesondere dessen bzw. deren Extrakt, miteinander kombiniert.

Gegenstand der vorliegenden Erfindung - gemäß einem **ersten** Aspekt der vorliegenden Erfindung - ist somit eine Zusammensetzung, insbesondere pharmazeutische Zubereitung in Form einer flüssigen Dosierung, insbesondere zur vorzugsweise topischen (lokalen) prophylaktischen oder therapeutischen (kurativen) Behandlung von insbesondere entzündlichen Erkrankungen des Mund- und Hals-/Rachenraums, wobei die Zusammensetzung in Kombination und in jeweils wirksamen, insbesondere pharmazeutisch wirksamen Mengen und jeweils bezogen auf die Zusammensetzung, enthält:
(a) 0,001 bis 30 Gew.-% mindestens einer Schleimdroge, wobei die Schleimdroge in Form eines Extraktes mit einem Droge/Extrakt-Verhältnis im Bereich von 0,4 : 1 bis 15 : 1 eingesetzt ist und wobei die Schleimdroge ausgewählt ist aus der Gruppe von Isländischem Moos (*Lichen islandicus*), Eibisch (*Althaea officinalis L*.), Spitzwegerich (*Plantago lanceolata L*.), Malve (*Malva sylvestris L.* und *M. neglecta WALLR*.), Boxhornklee (*Trigonella foenum-graecum L*.), Salep und/oder Quitte (*Cydonia oblonga MILL*.) sowie deren Kombinationen und Mischungen,
(b) 0,001 bis 10 Gew.-% Dexpanthenol (Panthenol, Pantothenol) und/oder Pantothensäure oder deren Derivate, insbesondere deren Ester oder Salze, und
(c) 0,001 bis 10 Gew.-% Holunder, insbesondere Holunderblüten, wobei die Zusammensetzung den Holunder, insbesondere die Holunderblüten, in Form eines Extraktes enthält.

Der Begriff "(pharmazeutische) Zusammensetzung" bzw. "(pharmazeutische) Kombination" - im Rahmen der vorliegenden Erfindung meist synonym verwendet - ist sehr weit zu verstehen und umfaßt jede Art von möglicher pharmazeutische Zusammensetzung oder Kombination, insbesondere Arzneimittel bzw. Pharmaka als solche, aber auch Medizinprodukte, homöopathische Mittel etc.

Bei allen nachstehend genannten relativen bzw. prozentualen gewichtsbezogenen Mengenangaben ist zu beachten, daß diese im Rahmen der erfindungsgemäßen Zusammensetzung derart auszuwählen sind, daß sie sich in der Summe in der erfindungsgemäßen Zusammensetzung unter Einbeziehung aller Komponenten bzw. Inhaltsstoffe bzw. Zusatzstoffe bzw. Bestandteile bzw. Exzipienten, insbesondere wie nachfolgend definiert, stets zu 100 Gew.-% ergänzen. Dies versteht sich für den Fachmann aber von selbst.

Im übrigen gilt, daß der Fachmann anwendungsbezogen oder einzelfallbedingt von den nachfolgend angeführten Mengenangaben abweichen kann, ohne daß er den Rahmen der vorliegenden Erfindung verläßt.

Es versteht sich zudem von selbst, daß Ausgestaltungen, Ausführungsformen, Vorteile und dergleichen, welche nachfolgend zu Zwecken der Vermeidung von Wiederholungen nur zu einem Erfindungsaspekt angeführt sind, selbstverständlich auch in bezug auf die übrigen Erfindungsaspekte entsprechend gelten.

Dies vorausgeschickt, wird die vorliegende Erfindung nun weiter im Detail erläutert.

Üblicherweise ist die erfindungsgemäße Zusammensetzung derart formuliert, daß sie die jeweiligen Wirk- und/oder Inhaltsstoffe zusammen mit einem unbedenklichen, insbesondere pharmakologisch und/oder physiologisch unbedenklichen, Träger und/oder Exzipienten enthält. Erfindungsgemäß ist letzterer flüssig bzw. fluid ausgebildet.

Wie zuvor geschildert, ist die vorliegende Erfindung auf Basis einer wenigstens ternären Mischung bzw. Kombination der drei vorgenannten Komponenten (a), (b) und (c) - im allgemeinen zusammen mit wenigstens einem Träger bzw. Exzipienten, wie zuvor definiert - ausgebildet.

Wie zuvor beschrieben, eignet sich die erfindungsgemäße Zusammensetzung insbesondere zur vorzugsweise topischen bzw. lokalen prophylaktischen oder therapeutischen (kurativen) Behandlung von insbesondere entzündlichen Erkrankungen des Mund- und Hals-/Rachenraums. Dabei wirkt die erfindungsgemäße Zusammensetzung insbesondere entzündungshemmend, reizlindernd sowie beruhigend auf die entzündeten Schleimhäute des Mund- und Hals-/Rachenraums. So eignet sich die erfindungsgemäße Zusammensetzung beispielsweise zur Befeuchtung im Hals- und Rachenbereich bei Schleimhautentzündungen, Halsschmerzen, Heiserkeit, rauhem Hals und belegter Stimme. Weiterhin kann die erfindungsgemäße Zusammensetzung beispielsweise bei gereizter Schleimhaut mit Halsschmerzen oder Halsschmerzen bei ausgetrockneter Schleimhaut oder bei trockener Rachenschleimhaut mit Halsschmerzen eingesetzt werden. Darüber hinaus kann die erfindungsgemäße Zusammensetzung bzw. Kombination gegen Halsschmerzen mit gleichzeitigem Schleimhautschutz eingesetzt werden.

Durch die erfindungsgemäße Kombination wird eine effiziente Mehrfachwirkung erreicht: Einerseits wird eine effiziente Wirksamkeit gegen die Entzündungsprozesse als solche bewirkt, und andererseits wird die Schleimhaut ausreichend benetzt, so daß einem Trockenheitsgefühl und einer Ausbreitung von Bakterien entgegengewirkt wird. Somit wirkt die erfindungsgemäße Zusammensetzung bzw. Kombination aktiv gegen Halsschmerzen und Mundtrockenheit und bekämpft gleichermaßen die Entzündung. Somit eignet sich die erfindungsgemä-βe Zusammensetzung beispielsweise für die unterstützende oder aber alleinige Behandlung beispielsweise bei Schleimhautreizung mit Halsschmerzen. Auf diese Weise werden Halsschmerzen effizient unter mehreren Gesichtspunkten bekämpft. Die Benetzung der ausgetrockneten Mundschleimhaut bewirkt zudem, daß das Eindringen von weiteren Erregern erschwert wird. Die Regeneration der entzündlich veränderten Mundschleimhaut kann in den von der erfindungsgemä-βen Zusammensetzung benetzten Bereichen ohne weiteres abheilen. Somit kommen effiziente Mehrfachwirkeffekte zum Tragen.

Die Anmelderin hat überraschenderweise herausgefunden, daß die erfindungsgemäße Kombination entzündungsbedingte Reizungen der Mund- und Rachenschleimhaut deutlich mindert, und zwar über die Wirkung der jeweiligen einzelnen Stoffe hinausgehend. Dies deutet auf einen synergistischen Effekt in bezug auf die erfindungsgemäße Kombination hin.

Im Rahmen der erfindungsgemäßen Zusammensetzung bewirkt die Schleimdroge bzw. deren Extrakt, insbesondere Isländisches Moos oder dessen Extrakt, daß ihre Inhaltsstoffe sich wie ein Schutzfilm auf die gereizten bzw. entzündeten Schleimhäute im Hals- und Rachenbereich legen, so daß auch äußere Reizeinwirkungen von den Schleimhäuten ferngehalten werden. Darüber hinaus besitzt die Schleimdroge, insbesondere Isländisches Moos, antiseptische bzw. desinfizierende Eigenschaften, welche als natürlicher Schutz gegen Erreger wirken. Holunder unterstützt den Wirkmechanismus der Schleimdroge; darüber hinaus wirkt der Holunder entzündungshemmend und stellt zudem wertvolle sekundäre Pflanzeninhaltsstoffe zur Verfügung. Dexpanthenol wiederum beruhigt die gereizten Schleimhäute und hilft ihnen bei der Regeneration.

Wie zuvor beschrieben, kommt als (Wirk-)Komponente (a) mindestens eine sogenannte Schleimdroge in Form ihres bevorzugt flüssigen bzw. fluiden Extraktes zum Einsatz.

Im Rahmen der vorliegenden Erfindung können eine Vielzahl von Schleimdrogen bzw. deren Extrakte zur Anwendung kommen. Erfindungsgemäß geeignete Schleimdrogen sind ausgewählt aus Isländischem Moos (*Lichen islandicus*), Eibisch (*Althaea oficinalis L*.), Spitzwegerich (*Plantago lanceolata L*.), Malve (*Malva sylvestris L.* und *M. neglecta WALLR*.), Boxhornklee (*Trigonella foenum-graecum L*.), Salep und/oder Quitte (*Cydonia oblonga MILL*.). Erfindungsgemäß bevorzugt sind Isländisches Moos (*Lichen islandicus*) und/oder Eibisch (*Althaea oficinalis L*.), und ganz besonders bevorzugt ist Isländisches Moos (*Lichen islandicus*); denn diese Schleimdrogen liefern im Rahmen der vorliegenden Erfindung die besten Ergebnisse.

Die einzelnen Schleimdrogen bzw. deren Extrakte sind als solche dem Fachmann hinlänglich bekannt:

Schleimdrogen werden wegen ihrer antiphlogistischen Eigenschaften äußerlich zur Behandlung von Furunkeln, Geschwüren, Drüsenschwellungen und Entzündungen des Rachenraumes, innerlich auch als Laxans, Antidiarrhoikum und zur Behandlung von Magen- und Darmentzündungen verwendet.

Schleime sind Heteropolysaccharide mit Molekulargewichten von ca. 50.000 bis 2.000.000, die durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten.

Isländisches Moos ist der deutsche Name für *Lichen islandicus* oder *Cetraria islandica* (*Parmeliaceae*), einer Flechte - entgegen dem deutschen Namen kein Moos -, die aus nördlichen Ländern, wie beispielsweise von Island, Norwegen und Schweden, exportiert wird. Isländisches Moos im eigentlichen Sinne besteht aus dem getrockneten Thallus von *Cetraria islandica* sowie dessen Zubereitungen. Die Droge enthält unter anderem Schleimstoffe und Bitterstoffe sowie bitterschmeckende Flechtensäuren. Aus der gepulverten Flechte lösen sich etwa 60 Gew.-% beim Kochen mit stark verdünnter Natriumhydrogencarbonatlösung, und beim Abkühlen der Lösung entsteht eine Gallerte. Der Extrakt besteht aus einem Polysaccharidgemisch von Lichenin und Isolichenin, einer Reihe von bitterschmeckenden Flechtensäuren (Fumarprotocetrar-, Protocetrar- und Cetrarsäure) sowie Protolichesterinsäure, die sich bei der Aufarbeitung in Lichesterinsäure umwandelt, und Usninsäure als antibiotisch wirkendem Flechtenfarbstoff. Als Schleimdroge besitzt Isländisches Moos reizlindemde Eigenschaften, es wirkt ebenso antimikrobiell. Der Bitterstoffgehalt begründet seine Anwendung bei Appetitlosigkeit. Isländisches Moos wird beispielsweise bei Katarrhen und Durchfall, als Bittertonikum, äußerlich bei schlecht heilenden Wunden, bei kosmetischen Präparaten, in Haarfixiermitteln, als Zusatz zu Biskuitmehl und dergleichen, bei Appetitlosigkeit sowie bei Schleimhautreizungen im Mund- und Rachenraum angewandt. Ein Mund- und Rachendesinfizienz auf Basis von Isländischem Moos wirkt reizlindernd bei trockenem Reizhusten. Das Kaltmazerat und andere bitterschmeckende Zubereitungen der Droge werden zur Behebung der Sub- oder Antacidität eingesetzt. Als Darreichungsformen werden je nach Anwendungsgebiet die zerkleinerte Droge für Aufgüsse sowie andere galenische Zubereitungen bzw. die zerkleinerte Droge vorzugsweise für Kaltmazerate sowie andere bitterschmeckende Zubereitungen zum Einnehmen angewandt. Für weitergehende Einzelheiten zu Isländischem Moos kann beispielsweise auf die folgende Literaturstellen verwiesen werden: Römpp Chemie-Lexikon, 9. Auflage, Band 3, Georg Thieme Verlag, Stuttgart/New York, 1990, Seiten 2055/2056, Stichwort: "Isländisches Moos"; HagerROM 2001, Springer Verlag, Heidelberg, Stichworte: "Cetraria", "Cetraria ericetorum OPIZ", "Cetraria islandica (L.) ACHARIUS" und "Lichen islandicus (Isländisches Moos)"; Monographie "Lichen islandicus (Isländisches Moos)", Bundesanzeiger Nr. 43 vom 2. März 1989; H. Wagner, "Pharmazeutische Biologie - Drogen und ihre Inhaltsstoffe", Gustav Fischer Verlag, Stuttgart/New York, 1985, Seite 281, Stichwort: "Cetrariae Lichen (= Lichen islandicus - Isländisches Moos)".

Der Eibisch (*Althaea oficinalis L*.), eingesetzt in Form der Eibischwurzel, -blätter und -blüten, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälte Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wäßrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirupus Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Der Spitzwegerich (*Plantago lanceolata L*.) ist in ganz Europa sowie in Nord- und Mittelasien verbreitet. Die Droge stammt von wild wachsenden Pflanzen und Kulturen vor allem Südosteuropas. Die getrockneten, oliv- bis bräunlichgrün gefärbten, lanzettlichen Blattspreiten mit etwa drei bis sieben parallel verlaufenden Nerven können verwendet werden. Die Herba-Droge besteht aus den getrockneten Blättern, Stengeln und ganzen Blüten. Neben dem Schleim enthält der Spitzwegerich als weitere Inhaltsstoffe Tannine, das Iridoidglykosid Aucubin (1,9 bis 2,4 %), das für die Dunkelfärbung einer nicht sorgfältig getrockneten Droge verantwortlich ist und zur Identitätsprüfung herangezogen wird, ferner das Senföl Sulforaphen. Der Spitzwegerich, insbesondere das Spitzwegerichblätter-Kraut, findet insbesondere Anwendung in Form von Tees und Sirupen bei Entzündungen des Mund- und Rachenraums.

Die Malve (*Malva sylvestris L.* und *M. neglecta WALLR*.), insbesondere eingesetzt in Form von Malvenblüten und -blättern, ist in Europa, Kleinasien, im Mittelmeergebiet und in Vorderindien heimisch. Zur Anwendung kommen die zur Blütezeit gesammelten, rosa-violett gefärbten Blüten bzw. die durch das Trocknen stark eingeschrumpften, gefalteten Blätter, die zumeist in Paketform in den Handel kommen. Die Malve ist ein zwei- bis mehrjähriges Kraut. Der Schleimgehalt von Blüten und Blättern liegt bei 6 bis 8 %. Bei der Hydrolyse liefert der Schleim Glucose, Arabinose, Rhamnose und Galactose. Malvenblüten werden zu Gurgelwässern und Bädern und zusammen mit den Blättern in Teemischungen als Expectorans verwendet.

Der Bockshornklee (*Trigonella foenum-graecum L*.), insbesondere eingesetzt in Form des Bockshomkleesamens, ist im gesamten Mittelmeergebiet, sowie in Osteuropa, Indien und China beheimatet. Der Hauptdrogenimport stammt aus den Kulturen Indiens und Marokkos. Zur Anwendung kommen die braun-rötlich gefärbten, vierseitigen und rautenförmigen, etwa 5 mm langen und etwa 3 mm breiten, flachgedrückten, sehr harten Samen, die durch eine Furche charakterisiert sind. Die Droge enthält außer fettem Öl 20 bis 30 % Schleim, Trigonellin, Nicotinsäureamid, Cholin, Bitterstoffe und Saponine. Die Herstellung des Schleims erfolgt aus dem gepulvertem Samen. Das Pulver wird angewendet äu-βerlich zu Umschlägen bei Furunkeln, Geschwüren und Drüsenschwellungen, innerlich als Expectorans und Roborans.

Salep bzw. Salep Tuber, insbesondere eingesetzt in Form der Salepknolle, ist von verschiedenen Orchideen-Arten abgeleitet, besonders *Orchis mascula L., Orchis morio L., Orchis militaris L., Anacamptis pyramidalis L. RICH.* und *Platanthera bifolia L. RICH.* Zur Anwendung kommen die etwa 4 cm langen bis etwa 3 cm dicken, runzeligen, harten, bräunlichen Knollen, die nach der Ernte von der Korbschicht befreit, mit siedendem Wasser abgebrüht und bei künstlicher Wärme getrocknet werden. Salepschleim, der bis zu 50 % vorwiegend aus Glucomannan bzw. Glucan besteht, wird hauptsächlich in der Kinderheilpraxis als Antidiarrhoikum verwendet.

Die Quitte (*Cydonia oblonga MILL*.) ist im südöstlichen Arabien heimisch. Hautdrogenimporte kommen aus Spanien, Portugal und Persien. Zur Anwendung kommen die reifen, getrockneten, rot-braun bis braun-violett gefärbten, etwas abgeplatteten Samen der Quittenscheinfrucht. Die Samen sind außen zum Teil mit einer eingetrockneten Schleimkruste versehen und oft miteinander verklebt. Sie besitzen einen schwachen Bittermandelgeschmack. In der Epidermis findet sich ca. 22 % Schleim, der zum größten Teil wasserlöslich ist. Die Zuckerkomponenten sind Arabinose, Xylose und Uronsäure, die zum Teil methyliert ist. Die Droge kommt nur unzerkleinert zur Anwendung. Der Quittenschleim wird äußerlich bei Lippen- und Brustwarzenrhagaden, bei Verbrennungen und Dekubitus in Salben- oder Cremeform verwendet.

Erfindungsgemäß besonders gute Ergebnisse werden mit Isländischem Moos bzw. dessen Extrakt erzielt.

Für weitergehende Einzelheiten zu Schleimdrogen und ihren Zubereitungen, Wirkungen und Anwendungen kann verwiesen werden auf H. Wagner "Pharmazeutische Biologie - Drogen und ihre Inhaltsstoffe", Gustav Fischer Verlag, Stuttgart/New York, 1985, insbesondere Seiten 280 ff.

Was die erfindungsgemäß eingesetzte Schleimdroge anbelangt, so wird diese erfindungsgemäß im allgemeinen in Form eines Extraktes, insbesondere eines Flüssigextraktes (Fluidextraktes), zugesetzt. Ein solcher Flüssigextrakt (Fluidextrakt) ist vorteilhafterweise auf wäßriger, alkoholischer oder wäßrig-alkoholischer Basis ausgebildet. Das Droge/Extrakt-Verhältnis kann dabei in weiten Bereichen variieren; wenn die Schleimdroge in Form eines Extraktes zugesetzt ist, wird insbesondere ein Droge/Extrakt-Verhältnis im Bereich von 0,4 : 1 bis 15:1, insbesondere im Bereich von 0,5 : 1 bis 10 : 1, bevorzugt im Bereich von 0,6 : 1 bis 8 : 1, besonders bevorzugt im Bereich von 0,8 : 1 bis 5 : 1, ganz besonders bevorzugt im Bereich von 0,9 : 1 bis 3 : 1, noch mehr bevorzugt von etwa 1 : 1, verwendet.

Besonders herausragende therapeutische Ergebnisse werden erzielt, wenn die Schleimdroge als Extrakt, vorzugsweise als Flüssigextrakt (Fluidextrakt), mit einem Droge/Extrakt-Verhältnis von mindestens 0,5 : 1, insbesondere mindestens 0,6 : 1, bevorzugt mindestens 0,8 : 1, besonders bevorzugt mindestens 0,9 : 1 oder mehr, zugesetzt ist.

Die Verwendung eines Extraktes einer Schleimdroge bietet gegenüber der Verwendung der Droge als solcher den entscheidenden Vorteil, daß hohe Konzentrationen Schleimdroge zur Anwendung kommen, so daß eine besonders gute therapeutische Wirkung erzielt wird.

Was die Menge an Schleimdroge(n) und/oder deren Extrakt(en) in der erfindungsgemäßen pharmazeutischen Zusammensetzung bzw. Kombination anbelangt, so kann die Menge in weiten Bereichen variieren. Im allgemeinen liegt die Menge an Schleimdroge(n) und/oder deren Extrakt(en) im Bereich von 0,01 bis 30 Gew.-%, insbesondere 0,1 bis 20 Gew.-%, bevorzugt 1 bis 15 Gew.-%, besonders bevorzugt 8 bis 12,5 Gew.-%, bezogen auf die erfindungsgemäße Zusammensetzung bzw. Kombination. Dennoch kann es anwendungsbedingt gegebenenfalls vorteilhaft oder erforderlich sein, von den vorgenannten Mengenangaben abzuweichen.

Weiterhin enthält die erfindungsgemäße Zusammensetzung als Komponente (b) Dexpanthenol (synonym auch als Panthenol oder Pantothenol bezeichnet) und/oder Pantothensäure oder deren Derivate, insbesondere Ester oder Salze, vorzugsweise Dexpanthenol.

Der Wirkstoff Dexpanthenol leitet sich von der Pantothensäure ab, welche ein Vitamin aus der Gruppe der B-Vitamine (Vitamin B₅) ist. Dexpanthenol ist besonders unter dem Handelsnamen Bepanthen^{®} bekannt. Pantothensäure ist ein Bestandteil des Coenzyms A und spielt damit eine wesentliche Rolle im Haut-und Schleimhautstoffwechsel. Im Rahmen der erfindungsgemäßen Zusammensetzung erhöht Dexpanthenol das Feuchthaltevermögen und hat somit pflegende Eigenschaften und verbessert die Elastizität der Schleimhaut. Es unterstützt die Neubildung der Schleimhautzellen und trägt so zu deren Regeneration bei. Darüber hinaus hat Dexpanthenol auch entzündungshemmende Eigenschaften. Im Rahmen der erfindungsgemäßen Zusammensetzung entfaltet somit das Dexpanthenol wundheilungsfördernde wie schleimhautschützende und darüber hinaus entzündungshemmende Eigenschaften.

Die Mengen an Dexpanthenol in der erfindungsgemäßen Zusammensetzung können gleichermaßen in weiten Bereichen variieren. Im allgemeinen enthält die erfindungsgemäße Zusammensetzung das Dexpanthenol und/oder die Pantothensäure bzw. deren Derivate in Mengen von 0,001 bis 10 Gew.-%, insbesondere 0,01 bis 7,5 Gew.-%, bevorzugt 0,1 bis 6 Gew.-%, besonders bevorzugt 1 bis 5 Gew.-%, bezogen auf die Zusammensetzung.

Des weiteren enthält die erfindungsgemäße Zusammensetzung als Wirkkomponente (c) Holunder, insbesondere Holunderblüten, und zwar in Form eines vorzugsweise flüssigen Extraktes, vorzugsweise Holunderblütenextraktes.

Die Holunder (*Sambucus*) bilden eine Gattung in der Familie der Moschuskrautgewächse (*Adoxaceae*). Die Gattung enthält weltweit etwa zwanzig bis dreißig Arten von denen drei in Mitteleuropa heimisch sind. Am bekanntesten von diesen drei Arten ist der Schwarze Holunder, der im heutigen Sprachgebrauch meist verkürzt als Holunder bezeichnet wird. Daneben gibt es den ebenfalls strauchförmigen Roten Holunder und den staudenförmigen Attich oder Zwergholunder. Die Arten wachsen im gemäßigten bis subtropischen Klima und in höheren Lagen von tropischen Gebirgen.

Wirkstoffe der Holunderblüten sind insbesondere ätherische Öle und Flavonoide sowie organische Säuren, Schleimstoffe und Gerbstoffe. Die Inhaltsstoffe des Holunders wirken stark schweißtreibend und stärkend auf die allgemeinen Abwehrkräfte (Immunsystem). Außerdem kann eine schleimlösende Wirkung beobachtet werden. Im Rahmen der erfindungsgemäßen Kombination bzw. Zusammensetzung unterstützt somit der Holunder die Wirkung der Schleimdroge und entfaltet darüber hinaus einen eigenen antiinflammatorischen Effekt und stellt darüber hinaus wertvolle sekundäre Pflanzeninhaltsstoffe bereit.

Die Mengen an Holunder, vorzugsweise Holunderblüten, insbesondere in Form des Extraktes, vorzugsweise Holunderblütenextraktes, kann gleichermaßen in weiteren Mengen variieren. Insbesondere enthält die Zusammensetzung Holunder, vorzugsweise Holunderblüten, insbesondere in Form eines Extraktes, vorzugsweise Holunderblütenextraktes, in Mengen von 0,001 bis 10 Gew.-%, insbesondere 0,005 bis 5 Gew.-%, bevorzugt 0,01 bis 2 Gew.-%, besonders bevorzugt 0,05 bis 1 Gew.-%, bezogen auf die erfindungsgemäße Zusammensetzung.

Weiterhin kann die erfindungsgemäße Zusammensetzung als Wirkkomponente (d) außerdem mindestens ein ätherisches Öl, vorzugsweise Pfefferminzöl, enthalten, insbesondere in Mengen von 0,001 bis 10 Gew.-%, insbesondere 0,01 bis 5 Gew.-%, bevorzugt 0,05 bis 2 Gew.-%, besonders bevorzugt 0,1 bis 1 Gew.-%, bezogen auf die erfindungsgemäße Zusammensetzung. Das ätherische Öl, insbesondere Pfefferminzöl unterstützt in wirkungsvoller Weise die Wirkeffizienz der übrigen Wirkkomponenten (a) bis (c).

Ätherische Öle sind ölige, leicht verdampfende Extrakte aus Pflanzen oder Pflanzenteilen, die einen starken, für die Herkunftspflanze charakteristischen Geruch haben. Im Gegensatz zu fetten Ölen verdampfen die ätherischen Öle vollständig und hinterlassen auf Papier keine Fettflecken. Sie sind aus vielen verschiedenen Komponenten zusammengesetzt, sind fettlöslich, enthalten jedoch keine Fette. In Wasser sind sie nur sehr wenig löslich; sie sind weniger dicht als Wasser und bilden daher schwimmende Flüssigkeitstropfen auf der Wasseroberfläche. Im Rahmen der vorliegenden Erfindung werden vorzugsweise ätherische Öle mit schleimlösender Wirkung eingesetzt.

In erfindungsgemäß bevorzugter Weise wird als ätherisches Öl Pfefferminzöl eingesetzt. Pfefferminzöl wird durch Wasserdampfdestillation von Pfefferminze (*Mentha piperita,* Lamiaceae) gewonnen. Hauptinhaltsstoffe des Pfefferminzöls sind das Menthol (35 bis 45 Gew.-%) und das Menthon (15 bis 20 Gew.-%), daneben noch Menthylacetat (3 bis 5 Gew.-%), Neomenthol (2,5 bis 3,5 Gew.-%) und Isomenthol (3 Gew.-%). Pfefferminzöl hat eine Dichte von 892 bis 927 g/l. Pfefferminzöl wirkt schwach antibakteriell und antifungal. Weiterhin hat es aromatisierende Wirkung.

Darüber hinaus kann die erfindungsgemäße Zusammensetzung als weitere Wirkkomponente (e) Natriumchlorid enthalten, insbesondere in Mengen von 0,01 bis 10 Gew.-%, insbesondere 0,05 bis 5 Gew.-%, bevorzugt 0,1 bis 2 Gew.-%, besonders bevorzugt 0,1 bis 1 Gew.-%, bezogen auf die erfindungsgemäße Zusammensetzung. Hierdurch wird die Feuchtigkeitsbildung der entzündeten bzw. gereizten Schleimhäuten angeregt, so daß hierdurch die Wirkeffizienz der übrigen Wirkkomponenten in sinnvoller Weise verstärkt wird.

Weiterhin kann die erfindungsgemäße Zusammensetzung als Wirkkomponente (f) Menthol enthalten, insbesondere in Mengen von 0,001 bis 5 Gew.-%, insbesondere 0,01 bis 2 Gew.-%, bevorzugt 0,02 bis 1 Gew.-%, besonders bevorzugt 0,05 bis 0, 1 Gew.-%, bezogen auf die erfindungsgemäße Zusammensetzung.

Menthol ist ein monocyclischer Monoterpen-Alkohol. Menthol wirkt schwach lokalanästhetisch und darüber hinaus analgetisch. Darüber hinaus findet es als Duft- und Aromastoff Verwendung.

Darüber hinaus kann die erfindungsgemäße Zusammensetzung mindestens einen weiteren Wirk-, Hilfs-, Additiv- und/oder Inhaltsstoff enthalten. Dieser kann beispielsweise ausgewählt sein aus der Gruppe von Verarbeitungshilfsmitteln, Aromastoffen, Geschmacksstoffen, Süßstoffen und Süßungsmitteln, Säuerungsmitteln, Stabilisatoren, Lokalanästhetika, Vitaminen, Mineralien, Spurenelementen und/oder Antiseptika sowie deren Mischungen oder Kombinationen.

Wie zuvor ausgeführt, liegt die erfindungsgemäße Zusammensetzung vorzugsweise in Form einer flüssigen Dosierung, vorzugsweise mit Eignung für die Sprühapplikation, vor.

Dabei kann die erfindungsgemäße Zusammensetzung vorteilhafterweise in einem Applikator für die Sprühapplikation eingebracht sein.

Gemäß einer besonderen Ausführungsform betrifft die vorliegenden Erfindung eine Zusammensetzung, insbesondere pharmazeutische Zubereitung in Form einer flüssigen Dosierung, welche sich insbesondere zur vorzugsweise topischen bzw. lokalen prophylaktischen oder therapeutischen (kurativen) Behandlung von insbesondere entzündlichen Erkrankungen des Mund- und Hals-/Rachenraums eignet, wie zuvor beschrieben, wobei die Zusammensetzung in Kombination und in jeweils wirksamen, insbesondere pharmazeutisch wirksamen Mengen, gegebenenfalls zusammen mit einem unbedenklichen, insbesondere pharmakologisch und/oder physiologisch unbedenklichen, Träger und/oder Exzipienten,
(a) mindestens eine Schleimdroge in Form eines Extraktes, insbesondere eines Flüssigextraktes (Fluidextraktes), aus der Gruppe von Isländischem Moos (*Lichen islandicus*), Eibisch (*Althaea officinalis L*.), Spitzwegerich (*Plantago lanceolata L*.), Malve (*Malva sylvestris L.* und *M. neglecta WALLR*.), Boxhornklee (*Trigonella foenum-graecum L*.), Salep und/oder Quitte (*Cydonia oblonga MILL*.) sowie deren Kombinationen und Mischungen und besonders bevorzugt Isländischem Moos (*Lichen islandicus*), in Mengen von 0,01 bis 30 Gew.-%, insbesondere 0,1 bis 20 Gew.-%, bevorzugt 1 bis 15 Gew.-%, besonders bevorzugt 8 bis 12,5 Gew.-%,
(b) Dexpanthenol (Panthenol, Pantothenol) und/oder Pantothensäure oder deren Derivate, insbesondere Ester oder Salze, in Mengen von 0,001 bis 10 Gew.-%, insbesondere 0,01 bis 7,5 Gew.-%, bevorzugt 0,1 bis 6 Gew.-%, besonders bevorzugt 1 bis 5 Gew.-%,
(c) Holunder, insbesondere Holunderblüten, in Form eines vorzugsweise flüssigen Extraktes, vorzugsweise Holunderblütenextraktes, in Mengen von 0,001 bis 10 Gew.-%, insbesondere 0,005 bis 5 Gew.-%, bevorzugt 0,01 bis 2 Gew.-%, besonders bevorzugt 0,05 bis 1 Gew.-%,
(d) gegebenenfalls mindestens ein ätherisches Öl, vorzugsweise Pfefferminzöl, insbesondere in Mengen von 0,001 bis 10 Gew.-%, insbesondere 0,01 bis 5 Gew.-%, bevorzugt 0,05 bis 2 Gew.-%, besonders bevorzugt 0,1 bis 1 Gew.-%,
(e) gegebenenfalls Natriumchlorid, insbesondere in Mengen von 0,01 bis 10 Gew.-%, insbesondere 0,05 bis 5 Gew.-%, bevorzugt 0,1 bis 2 Gew.-%, besonders bevorzugt 0,1 bis 1 Gew.-%,
(f) gegebenenfalls Menthol, insbesondere in Mengen von 0,001 bis 5 Gew.-%, insbesondere 0,01 bis 2 Gew.-%, bevorzugt 0,02 bis 1 Gew.-%, besonders bevorzugt 0,05 bis 0,1 1 Gew.-%,
   enthält, wobei alle vorgenannten Mengenangaben auf die Zusammensetzung bezogen sind.

Weiterer Gegenstand der vorliegenden Erfindung - gemäß einem **zweiten** erfindungsgemäßen Aspekt - ist ein Applikator, welcher sich vorzugsweise für die Sprühapplikation eignet und eine Zusammensetzung nach der vorliegenden Erfindung, wie sie zuvor beschrieben worden ist, enthält, insbesondere in Mengen von 10 bis 500 ml, vorzugsweise 25 bis 250 ml, bevorzugt 30 bis 150 ml. Für weitergehende Einzelheiten in bezug auf diesen erfindungsgemäßen Aspekt kann auf die obigen Ausführungen zu der erfindungsgemäßen pharmazeutischen Zusammensetzung bzw. Kombination verwiesen werden, die in bezug auf den erfindungsgemäßen Applikator entsprechend gelten.

Weiterer Gegenstand der vorliegenden Erfindung - gemäß einem **dritten** erfindungsgemäßen Aspekt - ist die Verwendung der erfindungsgemäßen Zusammensetzung zur vorzugsweise topischen (lokalen) prophylaktischen oder therapeutischen (kurativen) Behandlung von insbesondere entzündlichen Erkrankungen des Mund- und Hals-/Rachenraums bzw. zur Herstellung eines Arzneimittels zur vorzugsweise topischen (lokalen) prophylaktischen oder therapeutischen (kurativen) Behandlung von insbesondere entzündlichen Erkrankungen des Mund- und Hals-/Rachenraums.

Insbesondere läßt sich die erfindungsgemäße Zusammensetzung zur Behandlung von entzündlichen Erkrankungen des Mund- und Hals-/Rachenraums als Begleiterscheinung von Erkältungserkrankungen, grippalen Infekten oder dergleichen oder aber als eigenständige Erkrankungen einsetzen.

Weiterhin kann die erfindungsgemäße Zusammensetzung zur Behandlung von Halsschmerzen, Halsentzündungen, insbesondere Angina, Entzündungen des Kehlkopfs (Laryngitis), Entzündungen der Rachenschleimhaut (Pharyngitis), Entzündungen der Rachenmandeln (Tonsillitis), Entzündungen im Bereich der Mundhöhle, insbesondere Stomatitis, Gingivitis oder Mundschleimhautläsionen, oder dergleichen eingesetzt werden.

Des weiteren kann die erfindungsgemäße Zusammensetzung zur Befeuchtung im Hals- und Rachenbereich bei Schleimhautentzündungen, Halsschmerzen, Heiserkeit, rauhem Hals und belegter Stimme oder bei gereizter Schleimhaut mit Halsschmerzen oder bei Halsschmerzen mit ausgetrockneter Schleimhaut oder bei trockener Rachenschleimhaut mit Halsschmerzen oder zum Schleimhautschutz eingesetzt werden.

Üblicherweise wird die Zusammensetzung in Form einer flüssigen Dosierungsform, vorzugsweise im Sprühauftrag, appliziert, vorzugsweise mehrmals täglich.

Die erfindungsgemäße Zusammensetzung wird dabei entweder als Monotherapie oder aber als begleitende Therapie eingesetzt.

Für weitergehende Einzelheiten in bezug auf die erfindungsgemäße Verwendung kann auf obige Ausführungen zu der erfindungsgemäßen pharmazeutischen Zusammensetzung bzw. Kombination verwiesen werden, die in bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die folgenden Ausführungsbeispiele dienen lediglich der Veranschaulichung der vorliegenden Erfindung.

### AUSFÜHRUNGSBEISPIELE:

Es wurde eine erfindungsgemäße Zusammensetzung in Form einer flüssigen Dosierung zu Zwecken des Sprühauftrags mit den folgenden Komponenten hergestellt:

| | |
|---|---|
| *Lichen islandicus* (Fluidextrakt, 1:1) | 10,00 Gew.-% |
| Dexpanthenol | 2,75 Gew.-% |
| Holunderblütenextrakt | 0,20 Gew.-% |
| Natriumchlorid | 0,78 Gew.-% |
| Pfefferminzöl | 0,48 Gew.-% |
| Menthol | 0,07 Gew.-% |
| Hilfsstoffe (Aromen, Konservierungsmittel Süßungsmittel) | 0,81 Gew.-% |
| gereinigtes Wasser | ad 100,00 Gew.-% |

Des weiteren wurden drei Vergleichszusammensetzungen hergestellt, in denen jeweils der Anteil an *Lichen islandicus* (Vergleichszusammensetzung 1) bzw. Dexpanthenol (Vergleichszusammensetzung 2) bzw. Holunderblütenextrakt (Vergleichszusammensetzung 3) durch den gleichen Anteil an Wasser ersetzt wurde.

20 Patienten mit akuten schmerzhaften Halsentzündungen infolge von Erkältungserkrankungen, einhergehend mit Heiserkeit und trockenem Hals, wurden ohne zusätzliche systemische Therapie ausschließlich topisch mit einer flüssigen Dosierung im Sprühauftrag therapiert.

Bei 5 Patienten wurde die zuvor beschriebene erfindungsgemäße Zusammensetzung eingesetzt, bei jeweils weiteren 5 Patienten die Vergleichszusammensetzung 1 bzw. 2 bzw. 3.

Bei allen Präparaten zeigte sich eine lindernde und darüber hinaus entzündungshemmende Wirkung. Bei den mit der erfindungsgemäßen Zusammensetzung behandelten Patienten trat bereits nach ein bis drei Tagen eine deutliche Linderung der Entzündungen auf und waren diese Entzündungen bereits innerhalb von fünf bis acht Tagen vollständig abgeklungen, während dies bei den nichterfindungsgemäßen Zusammensetzungen deutlich länger andauerte: Bei der Behandlung mit den drei nichterfindungsgemäßen Zusammensetzungen traten erste Linderungen erst nach etwa vier bis fünf Tagen auf und waren die letzten Symptome erst nach mehr als zehn Tagen Behandlungsdauer verschwunden.

Die Untersuchungen zeigen, daß die Anwendung der erfindungsgemäßen Zusammensetzung in Form einer ternären Kombination gegenüber den nichterfindungsgemäßen Anwendungsformen einen deutlich gesteigerten Therapieerfolg bewirkt. Hierin liegt ein entscheidender Vorteil der erfindungsgemäßen Zusammensetzung.

## Patentansprüche

1. Zusammensetzung, insbesondere pharmazeutische Zubereitung in Form einer flüssigen Dosierung, insbesondere zur vorzugsweise topischen (lokalen) prophylaktischen oder therapeutischen (kurativen) Behandlung von insbesondere entzündlichen Erkrankungen des Mund- und Hals-/Rachenraums, wobei die Zusammensetzung in Kombination und in jeweils wirksamen, insbesondere pharmazeutisch wirksamen Mengen und jeweils bezogen auf die Zusammensetzung, enthält:
(a) 0,001 bis 30 Gew.-% mindestens einer Schleimdroge,
wobei die Schleimdroge in Form eines Extraktes mit einem Droge/Extrakt-Verhältnis im Bereich von 0,4 : 1 bis 15 : 1 eingesetzt ist und wobei die Schleimdroge ausgewählt ist aus der Gruppe von Isländischem Moos (*Lichen islandicus*), Eibisch (*Althaea officinalis L*.), Spitzwegerich (*Plantago lanceolata L*.), Malve (*Malva sylvestris L.* und *M. neglecta WALLR*.), Boxhornklee (*Trigonella foenum-graecum L*.), Salep und/oder Quitte (*Cydonia oblonga MILL*.) sowie deren Kombinationen und Mischungen,
(b) 0,001 bis 10 Gew.-% Dexpanthenol (Panthenol, Pantothenol) und/oder Pantothensäure oder deren Derivate, insbesondere deren Ester oder Salze, und
(c) 0,001 bis 10 Gew.-% Holunder, insbesondere Holunderblüten,
wobei die Zusammensetzung den Holunder, insbesondere die Holunderblüten, in Form eines Extraktes enthält.

2. Zusammensetzung nach Anspruch 1, wobei die Schleimdroge in Form eines Flüssigextraktes (Fluidexträktes) eingesetzt ist und/oder wobei die Schleimdroge in Form eines wäßrigen, alkoholischen oder wäßrig-alkoholischen Extrakts eingesetzt ist.

3. Zusammensetzung nach Anspruch 1 oder 2, wobei die Schleimdroge in Form eines Extraktes, insbesondere Flüssigextraktes, mit einem Droge/Extrakt-Verhältnis im Bereich von 0,5 : 1 bis 10 : 1, bevorzugt im Bereich von 0,6 : 1 bis 8 : 1, besonders bevorzugt im Bereich von 0,8 : 1 bis 5 : 1, ganz besonders bevorzugt im Bereich von 0,9 : 1 bis 3 : 1, noch mehr bevorzugt von etwa 1 : 1, zugesetzt ist.

4. Zusammensetzung nach einem der vorangehenden Ansprüche, wobei die Schleimdroge ausgewählt ist aus Isländischem Moos (*Lichen islandicus*) und/oder Eibisch (*Althaea oficinalis L*.), besonders bevorzugt Isländischem Moos (*Lichen islandicus*).

5. Zusammensetzung nach einem der vorangehenden Ansprüche, wobei die Schleimdroge Isländisches Moos (*Lichen islandicus*) in Form dessen Extraktes ist.

6. Zusammensetzung nach einem der vorangehenden Ansprüche, wobei die Zusammensetzung die Schleimdroge in Form von deren Extrakt in Mengen 0,1 bis 20 Gew.-%, bevorzugt 1 bis 15 Gew.-%, besonders bevorzugt 8 bis 12,5 Gew.-%, bezogen auf die Zusammensetzung, enthält.

7. Zusammensetzung nach einem der vorangehenden Ansprüche, wobei die Zusammensetzung Dexpanthenol (Panthenol, Pantothenol) und/oder Pantothensäure oder deren Derivate, insbesondere Ester oder Salze, vorzugsweise Dexpanthenol, in Mengen von 0,01 bis 7,5 Gew.-%, bevorzugt 0,1 bis 6 Gew.-%, besonders bevorzugt 1 bis 5 Gew.-%, bezogen auf die Zusammensetzung, enthält.

8. Zusammensetzung nach einem der vorangehenden Ansprüche, wobei die Zusammensetzung Holunder, insbesondere Holunderblüten, in Form eines flüssigen Extraktes, vorzugsweise Holunderblütenextraktes, enthält.

9. Zusammensetzung nach einem der vorangehenden Ansprüche, wobei die Zusammensetzung Holunder, vorzugsweise Holunderblüten, in Form eines Extraktes, vorzugsweise Holunderblütenextraktes, in Mengen von 0,005 bis 5 Gew.-%, bevorzugt 0,01 bis 2 Gew.-%, besonders bevorzugt 0,05 bis 1 Gew.-%, bezogen auf die Zusammensetzung, enthält.

10. Zusammensetzung nach einem der vorangehenden Ansprüche, wobei die Zusammensetzung außerdem (d) mindestens ein ätherisches Öl, vorzugsweise Pfefferminzöl, enthält, insbesondere in Mengen von 0,001 bis 10 Gew.-%, insbesondere 0,01 bis 5 Gew.-%, bevorzugt 0,05 bis 2 Gew.-%, besonders bevorzugt 0,1 bis 1 Gew.-%, bezogen auf die Zusammensetzung, und/oder wobei die Zusammensetzung außerdem (e) Natriumchlorid enthält, insbesondere in Mengen von 0,01 bis 10 Gew.-%, insbesondere 0,05 bis 5 Gew.-%, bevorzugt 0,1 bis 2 Gew.-%, besonders bevorzugt 0,1 bis 1 Gew.-%, bezogen auf die Zusammensetzung, und/oder
wobei die Zusammensetzung außerdem (f) Menthol enthält, insbesondere in Mengen von 0,001 bis 5 Gew.-%, insbesondere 0,01 bis 2 Gew.-%, bevorzugt 0,02 bis 1 Gew.-%, besonders bevorzugt 0,05 bis 0,1 Gew.-%, bezogen auf die Zusammensetzung.

11. Zusammensetzung nach einem der vorangehenden Ansprüche, wobei die Zusammensetzung in Form einer flüssigen Dosierung, vorzugsweise für die Sprühapplikation, vorliegt.

12. Zusammensetzung, insbesondere pharmazeutische Zubereitung in Form einer flüssigen Dosierung, insbesondere zur vorzugsweise topischen (lokalen) prophylaktischen oder therapeutischen (kurativen) Behandlung von insbesondere entzündlichen Erkrankungen des Mund- und Hals-/Rachenraums, nach einem der vorangehenden Ansprüche, wobei die Zusammensetzung in Kombination und in jeweils wirksamen, insbesondere pharmazeutisch wirksamen Mengen, gegebenenfalls zusammen mit einem unbedenklichen, insbesondere pharmakologisch und/oder physiologisch unbedenklichen, Träger und/oder Exzipienten,
(a) mindestens eine Schleimdroge in Form eines Extraktes, insbesondere eines Flüssigextraktes (Fluidextraktes), aus der Gruppe von Isländischem Moos (*Lichen islandicus*), Eibisch (*Althaea officinalis L*.), Spitzwegerich (*Plantago lanceolata L*.), Malve (*Malva sylvestris L.* und *M. neglecta WALLR*.), Boxhornklee (*Trigonella foenum-graecum L*.), Salep und/oder Quitte (*Cydonia oblonga MILL*.) sowie deren Kombinationen und Mischungen und besonders bevorzugt Isländischem Moos (*Lichen islandicus*), in Mengen von 0,01 bis 30 Gew.-%, insbesondere 0,1 bis 20 Gew.-%, bevorzugt 1 bis 15 Gew.-%, besonders bevorzugt 8 bis 12,5 Gew.-%,
(b) Dexpanthenol (Panthenol, Pantothenol) und/oder Pantothensäure oder deren Derivate, insbesondere Ester oder Salze, in Mengen von 0,001 bis 10 Gew.-%, insbesondere 0,01 bis 7,5 Gew.-%, bevorzugt 0,1 bis 6 Gew.-%, besonders bevorzugt 1 bis 5 Gew.-%,
(c) Holunder, insbesondere Holunderblüten, in Form eines vorzugsweise flüssigen Extraktes, vorzugsweise Holunderblütenextraktes, in Mengen von 0,001 bis 10 Gew.-%, insbesondere 0,005 bis 5 Gew.-%, bevorzugt 0,01 bis 2 Gew.-%, besonders bevorzugt 0,05 bis 1 Gew.-%,
(d) gegebenenfalls mindestens ein ätherisches Öl, vorzugsweise Pfefferminzöl, insbesondere in Mengen von 0,001 bis 10 Gew.-%, insbesondere 0,01 bis 5 Gew.-%, bevorzugt 0,05 bis 2 Gew.-%, besonders bevorzugt 0,1 bis 1 Gew.-%,
(e) gegebenenfalls Natriumchlorid, insbesondere in Mengen von 0,01 bis 10 Gew.-%, insbesondere 0,05 bis 5 Gew.-%, bevorzugt 0,1 bis 2 Gew.-%, besonders bevorzugt 0,1 bis 1 Gew.-%,
(f) gegebenenfalls Menthol, insbesondere in Mengen von 0,001 bis 5 Gew.-%, insbesondere 0,01 bis 2 Gew.-%, bevorzugt 0,02 bis 1 Gew.-%, besonders bevorzugt 0,05 bis 0,1 Gew.-%,
enthält, wobei alle vorgenannten Mengenangaben auf die Zusammensetzung bezogen sind.

13. Applikator, vorzugsweise für die Sprühapplikation, enthaltend eine Zusammensetzung nach einem der vorangehenden Ansprüche, insbesondere in Mengen von 10 bis 500 ml, vorzugsweise 25 bis 250 ml, bevorzugt 30 bis 150 ml.

14. Verwendung einer Zusammensetzung nach einem der vorangehenden Ansprüche zur Herstellung eines Arzneimittels zur vorzugsweise topischen (lokalen) prophylaktischen oder therapeutischen (kurativen) Behandlung von insbesondere entzündlichen Erkrankungen des Mund- und Hals-/Rachenraums.

15. Verwendung nach Anspruch 14, wobei die Zusammensetzung in Form einer flüssigen Dosierungsform, vorzugsweise im Sprühauftrag, appliziert wird, vorzugsweise mehrmals täglich.

## Claims

1. A composition, in particular a pharmaceutical preparation in the form of a liquid dosage, in particular for the preferably topical (local) prophylactic or therapeutic (curative) treatment of particularly inflammable diseases of the mouth and throat/pharyngeal zone, wherein the composition comprises the following in combination and in effective, in particular pharmaceutically effective amounts, and each based on the composition:
(a) 0.001 % to 30 % by weight of at least one mucus drug,
wherein the mucus drug is used in the form of an extract having a drug/extract ratio within the range of 0.4:1 to 15:1, and
wherein the mucus drug is selected from the group of Icelandic moss (*Lichen islandicus*), marshmallow (*Althaea officinalis L*.), buckhorn plantain (*Plantago lanceolata L*.), mallow (*Malva sylvestris L.* and *M. neglecta WALLR*.), fenugreek (*Trigonella foenum-graecum L*.), salep and/or quine (*Cydonia oblonga MILL*.), as well as the combinations and mixtures thereof,
(b) 0.001 % to 10 % by weight of Dexpanthenol (panthenol, pantothenol) and/or pantothenic acid or the derivatives thereof, in particular the esters or salts thereof, and
(c) 0.001 % to 10 % by weight of elder, in particular elder blossoms, wherein the composition comprises the elder, in particular the elder blossoms, in the form of an extract.

2. The composition according to claim 1, wherein the mucus drug is used in the form of a liquid extract (fluid extract) and/or wherein the mucus drug is used in the form of an aqueous, alcoholic, or aqueous-alcoholic extract.

3. The composition according to claims 1 or 2, wherein the mucus drug is added in the form of an extract, in particular a liquid extract, at a drug/extract ratio within the range of 0.5:1 to 10:1, preferably within the range of 0.6:1 to 8:1, particularly preferred within the range of 0.8:1 to 5:1, more preferred within the range of 0.9:1 to 3:1, most preferred of about 1:1.

4. The composition according to one of the preceding claims, wherein the mucus drug is selected from Icelandic moss (*Lichen islandicus*) and/or marshmallow (*Althaea officinalis L*.), particularly preferred from Icelandic moss (*Lichen islandicus*).

5. The composition according to one of the preceding claims, wherein the mucus drug is Icelandic moss (*Lichen islandicus*) in the form of the extract thereof.

6. The composition according to one of the preceding claims, wherein the composition comprises the mucus drug in the form of the extract thereof in amounts of 0.1 % to 20 % by weight, preferably 1 % to 15 % by weight, particularly preferred of 8 % to 12.5 % by weight, based on the composition.

7. The composition according to one of the preceding claims, wherein the composition comprises Dexpanthenol (panthenol, pantothenol) and/or pantothenic acid or the derivatives thereof, in particular esters or salts, preferably Dexpanthenol, in amounts of 0.01 % to 7.5 % by weight, preferably 0.1 % to 6 % by weight, particularly preferred of 1 % to 5 % by weight, based on the composition.

8. The composition according to one of the preceding claims, wherein the composition comprises elder, in particular elder blossoms, in the form of a liquid extract, preferably an elder blossom extract.

9. The composition according to one of the preceding claims, wherein the composition comprises elder, in particular elder blossoms, in the form of a liquid extract, preferably an elder blossom extract, in amounts of 0.005 % to 5 % by weight, preferably 0.01 % to 2 % by weight, particularly preferred of 0.05 % to 1 % by weight, based on the composition.

10. The composition according to one of the preceding claims, the composition further comprising (d) at least one essential oil, preferably peppermint oil, in particular in amounts of 0.001 % to 10 % by weight, in particular 0.01 % to 5 % by weight, preferably of 0.05 % to 1 % by weight, particularly preferred of 0.1 % to 1 % by weight, based on the composition, and/or wherein the composition further comprises (e) sodium chloride, in particular in amounts of 0.01 % to 10 % by weight, in particular 0.05 % to 5 % by weight, preferably of 0.1 % to 2 % by weight, particularly preferred of 0.1 % to 1 % by weight, based on the composition, and/or
wherein the composition further comprises (f) menthol, in particular in amounts of 0.001 % to 5 % by weight, in particular 0.01 % to 2 % by weight, preferably of 0.02 % to 1 % by weight, particularly preferred of 0.05 % to 0.1 % by weight, based on the composition.

11. The composition according to one of the preceding claims, the composition is present in the form of a liquid dosage, preferably for a spray application.

12. The composition, in particular pharmaceutical preparation in the form of a liquid dosage, in particular for the preferred topical (local) prophylactic or therapeutic (curative) treatment of particularly inflammable diseases of the mouth and throat/pharyngeal zone, according to one of the preceding claims, wherein the composition comprises in combination and in effective, in particular pharmaceutically effective amounts, and where appropriate together with an acceptable, in particular pharmacologically and/or physiologically acceptable, carrier and/or excipient,
(a) at least one mucus drug in the form of an extract, in particular a liquid extract (fluid extract), from the group of Icelandic moss (*Lichen islandicus*), marshmallow (*Althaea officinalis L*.), buckhorn plantain (*Plantago lanceolata L*.), mallow (*Malva sylvestris L.* and *M. neglecta WALLR*.), fenugreek (*Trigonella foenum-graecum L*.), salep and/or quine (*Cydonia oblonga MILL*.), as well as the combinations and mixtures thereof, and particularly preferred Icelandic moss (*Lichen islandicus*), in amounts of 0.01 % to 30 % by weight, in particular 0.1 % to 20 % by weight, preferably of 1 % to 15 % by weight, particularly preferred of 8 % to 12.5 % by weight,
(b) Dexpanthenol (panthenol, pantothenol) and/or panthothenic acid or the derivatives thereof, in particular esters or salts, in amounts of 0.001 % to 10 % by weight, in particular 0.01 % to 7.5 % by weight, preferably of 0.1 % to 6 % by weight, particularly preferred of 1 % to 5 % by weight,
(c) elder, in particular elder blossoms, in the form of a preferably liquid extract, preferably elder blossom extract, in amounts of 0.001 % to 10 % by weight, in particular 0.005 % to 5 % by weight, preferably of 0.01 % to 2 % by weight, particularly preferred of 0.05 % to 1 % by weight,
(d) where appropriate, at least one essential oil, preferably peppermint oil, in particular in amounts of 0.001 % to 10 % by weight, in particular 0.01 % to 5 % by weight, preferably of 0.05 % to 2 % by weight, particularly preferred of 0.1 % to 1 % by weight,
(e) where appropriate, sodium chloride, in particular in amounts of 0.01 % to 10 % by weight, in particular 0.05 % to 5 % by weight, preferably of 0.1 % to 2 % by weight, particularly preferred of 0.1 % to 1 % by weight,
(f) where appropriate, menthol, in particular in amounts of 0.001 % to 5 % by weight, in particular 0.01 % to 2 % by weight, preferably of 0.02 % to 1 % by weight, particularly preferred of 0.05 % to 0.1 % by weight, wherein all amounts stated above are based on the composition.

13. An applicator, preferably for spray application, comprising a composition according to one of the preceding claims, in particular in amounts of 10 to 500 ml, preferably 25 to 250 ml, more preferred 30 to 150 ml.

14. A use of a composition according to one of the preceding claims for the production of a pharmaceutical for the preferably topical (local) prophylactic or therapeutic (curative) treatment of particularly inflammatory diseases of the mouth and throat/pharyngeal zone.

15. The use according to claim 14, wherein the composition is applied in the form of a liquid dosage form, preferably as a spray application, preferably multiple times per day.

## Revendications

1. Composition, notamment préparation pharmaceutique sous la forme d'une dose liquide, notamment pour le traitement prophylactique ou thérapeutique (curatif), de préférence topique (local), notamment de maladies inflammatoires de la cavité buccale et de la gorge/du pharynx, dans laquelle la composition contient en combinaison et à chaque fois en quantités efficaces, notamment pharmaceutiquement efficaces, et à chaque fois par rapport à la composition:
(a) 0,001 à 30 % en poids d'au moins une drogue mucilagineuse,
la drogue mucilagineuse étant utilisée sous la forme d'un extrait ayant un rapport drogue/extrait dans la plage allant de 0,4:1 à 15:1, et
la drogue mucilagineuse étant choisie dans le groupe constitué par la mousse d'Islande (*Lichen islandicus*), la guimauve (*Althaea officinalis L*.), le plantain lancéolé (*Plantago lanceolata L*.), la mauve (*Malva sylvestris L.* et *M. neglecta WALLR*.), le fénugrec (*Trigonella foenum-graecum L*.), le salep et/ou le coing (*Cydonia oblonga MILL*.), ainsi que leurs combinaisons et leurs mélanges,
(b) 0,001 à 10 % en poids de dexpanthénol (panthénol, pantothénol) et/ou d'acide pantothénique ou ses dérivés, notamment ses esters ou ses sels, et
(c) 0,001 à 10 % en poids de sureau, notamment de fleurs de sureau,
la composition contenant le sureau, notamment les fleurs de sureau, sous la forme d'un extrait.

2. Composition selon la revendication 1, dans laquelle la drogue mucilagineuse est utilisée sous la forme d'un extrait liquide (extrait fluide) et/ou dans laquelle la drogue mucilagineuse est utilisée sous la forme d'un extrait aqueux, alcoolique ou aqueux-alcoolique.

3. Composition selon la revendication 1 ou 2, dans laquelle la drogue mucilagineuse est ajoutée sous la forme d'un extrait, notamment d'un extrait liquide, ayant un rapport drogue/extrait dans la plage allant de 0,5:1 à 10:1, de préférence dans la plage allant de 0,6:1 à 8:1, de manière particulièrement préférée dans la plage allant de 0,8:1 à 5:1, de manière tout particulièrement préférée dans la plage allant de 0,9:1 à 3:1, de manière encore davantage préférée d'environ 1:1.

4. Composition selon l'une quelconque des revendications précédentes, dans laquelle la drogue mucilagineuse est choisie parmi la mousse d'Islande (*Lichen islandicus*) et/ou la guimauve (*Althaea officinalis L*.), de manière particulièrement préférée la mousse d'Islande (*Lichen islandicus*).

5. Composition selon l'une quelconque des revendications précédentes, dans laquelle la drogue mucilagineuse est la mousse d'Islande (*Lichen islandicus*) sous la forme de son extrait.

6. Composition selon l'une quelconque des revendications précédentes, dans laquelle la composition contient la drogue mucilagineuse sous la forme de son extrait en quantités de 0,1 à 20 % en poids, de préférence de 1 à 15 % en poids, de manière particulièrement préférée de 8 à 12,5 % en poids, par rapport à la composition.

7. Composition selon l'une quelconque des revendications précédentes, dans laquelle la composition contient le dexpanthénol (panthénol, pantothénol) et/ou l'acide pantothénique ou ses dérivés, notamment ses esters ou ses sels, de préférence le dexpanthénol, en quantités de 0,01 à 7,5 % en poids, de préférence de 0,1 à 6 % en poids, de manière particulièrement préférée de 1 à 5 % en poids, par rapport à la composition.

8. Composition selon l'une quelconque des revendications précédentes, dans laquelle la composition contient le sureau, notamment les fleurs de sureau, sous la forme d'un extrait liquide, de préférence d'un extrait de fleurs de sureau.

9. Composition selon l'une quelconque des revendications précédentes, dans laquelle la composition contient le sureau, notamment les fleurs de sureau, sous la forme d'un extrait, de préférence d'un extrait de fleurs de sureau, en quantités de 0,005 à 5 % en poids, de préférence de 0,01 à 2 % en poids, de manière particulièrement préférée de 0,05 à 1 % en poids, par rapport à la composition.

10. Composition selon l'une quelconque des revendications précédentes, dans laquelle la composition contient également (d) au moins une huile essentielle, de préférence de l'huile de menthe poivrée, notamment en quantités de 0,001 à 10 % en poids, notamment de 0,01 à 5 % en poids, de préférence de 0,05 à 2 % en poids, de manière particulièrement préférée de 0,1 à 1 % en poids, par rapport à la composition, et/ou
dans laquelle la composition contient également (e) du chlorure de sodium, notamment en quantités de 0,01 à 10 % en poids, notamment de 0,05 à 5 % en poids, de préférence de 0,1 à 2 % en poids, de manière particulièrement préférée de 0,1 à 1 % en poids, par rapport à la composition, et/ou dans laquelle la composition contient également (f) du menthol, notamment en quantités de 0,001 à 5 % en poids, notamment de 0,01 à 2 % en poids, de préférence de 0,02 à 1 % en poids, de manière particulièrement préférée de 0,05 à 0,1 % en poids, par rapport à la composition.

11. Composition selon l'une quelconque des revendications précédentes, dans laquelle la composition se présente sous la forme d'une dose liquide, de préférence pour l'application par pulvérisation.

12. Composition, notamment préparation pharmaceutique sous la forme d'une dose liquide, notamment pour le traitement prophylactique ou thérapeutique (curatif), de préférence topique (local), notamment de maladies inflammatoires de la cavité buccale et de la gorge/du pharynx, selon l'une quelconque des revendications précédentes, dans laquelle la composition contient en combinaison et à chaque fois en quantités efficaces, notamment pharmaceutiquement efficaces, éventuellement avec un véhicule inoffensif, notamment pharmacologiquement et/ou physiologiquement inoffensif, et/ou des excipients:
(a) au moins une drogue mucilagineuse sous la forme d'un extrait, notamment d'un extrait liquide (extrait fluide) du groupe constitué par la mousse d'Islande (*Lichen islandicus*), la guimauve (*Althaea officinalis L*.), le plantain lancéolé (*Plantago lanceolata L*.), la mauve (*Malva sylvestris L.* et *M. neglecta WALLR*.), le fénugrec (*Trigonella foenum-graecum L*.), le salep et/ou le coing (*Cydonia oblonga MILL*.), ainsi que leurs combinaisons et leurs mélanges, et de manière particulièrement préférée la mousse d'Islande (*Lichen islandicus*), en quantités de 0,01 à 30 % en poids, notamment de 0,1 à 20 % en poids, de préférence de 1 à 15 % en poids, de manière particulièrement préférée de 8 à 12,5 % en poids,
(b) du dexpanthénol (panthénol, pantothénol) et/ou de l'acide pantothénique ou ses dérivés, notamment ses esters ou ses sels, en quantités de 0,001 à 10 % en poids, notamment de 0,01 à 7,5 % en poids, de préférence de 0,1 à 6 % en poids, de manière particulièrement préférée de 1 à 5 % en poids,
(c) du sureau, notamment des fleurs de sureau, sous la forme d'un extrait de préférence liquide, de préférence d'un extrait de fleurs de sureau, en quantités de 0,001 à 10 % en poids, notamment de 0,005 à 5 % en poids, de préférence de 0,01 à 2 % en poids, de manière particulièrement préférée de 0,05 à 1 % en poids,
(d) éventuellement au moins une huile essentielle, de préférence de l'huile de menthe poivrée, notamment en quantités de 0,001 à 10 % en poids, notamment de 0,01 à 5 % en poids, de préférence de 0,05 à 2 % en poids, de manière particulièrement préférée de 0,1 à 1 % en poids,
(e) éventuellement du chlorure de sodium, notamment en quantités de 0,01 à 10 % en poids, notamment de 0,05 à 5 % en poids, de préférence de 0,1 à 2 % en poids, de manière particulièrement préférée de 0,1 à 1 % en poids,
(f) éventuellement du menthol, notamment en quantités de 0,001 à 5 % en poids, notamment de 0,01 à 2 % en poids, de préférence de 0,02 à 1 % en poids, de manière particulièrement préférée de 0,05 à 0,1 % en poids,
toutes les données de quantité susmentionnées se rapportant à la composition.

13. Applicateur, de préférence pour l'application par pulvérisation, contenant une composition selon l'une quelconque des revendications précédentes, notamment en quantités de 10 à 500 ml, avantageusement de 25 à 250 ml, de préférence de 30 à 150 ml.

14. Utilisation d'une composition selon l'une quelconque des revendications précédentes pour la fabrication d'un médicament pour le traitement prophylactique ou thérapeutique (curatif), de préférence topique (local), notamment de maladies inflammatoires de la cavité buccale et de la gorge/du pharynx.

15. Utilisation selon la revendication 14, dans laquelle la composition est appliquée sous la forme d'une forme posologique liquide, de préférence par application par pulvérisation, de préférence plusieurs fois par jour.
